# EUROPEAN PATENT SPECIFICATION

(11) **EP 2 027 260 B1**
(45) Date of publication and mention of the grant of the patent: **11.12.2013**
(21) Application number: 07732716.1
(22) Date of filing: 08.05.2007
(51) Int. Cl.: C07K 19/00

(54) **EPIGENETIC REGULATORY COMPLEX FOR CONTROL OF GENE EXPRESSION**
EPIGENETISCHER REGULATORISCHER KOMPLEX ZUR GENEXPRESSIONSKONTROLLE
COMPLEXE RÉGULATEUR ÉPIGÉNÉTIQUE POUR CONTRÔLER DE L'EXPRESSION GÉNIQUE

(30) Priority: 05.05.2006 GB 0608945; 05.05.2006 US 798029 P
(43) Date of publication of application: 25.02.2009
(73) Proprietor: Cambridge Enterprise Limited, Cambridge CB2 1TS (GB)
(72) Inventor: SURANI, Azim, Cambridge CB2 1QR (GB); LANGE, Ulrike, D-79110 Freiburg (DE); HAJKOVA, Petra, Cambridge CB2 1QR (GB); ANCELIN, Katia, 75013 Paris (FR)
(74) Representative: Crease, Devanand John
(86) International application number: PCT/GB2007/001688
(87) International publication number: WO 2007/129091

(56) References cited:
- US-A1- 2004 186 274
- VINCENT STEPHANE D ET AL: "The zinc finger transcriptional repressor Blimp1/Prdm1 is dispensable for early axis formation but is required for specification of primordial germ cells in the mouse" DEVELOPMENT (CAMBRIDGE), vol. 132, no. 6, March 2005 (2005-03), pages 1315-1325, XP002452828 ISSN: 0950-1991
- RICHARD STEPHANE ET AL: "Arginine methylation regulates IL-2 gene expression: a role for protein arginine methyltransferase 5 (PRMT5)" BIOCHEMICAL JOURNAL, vol. 388, no. Part 1, May 2005 (2005-05), pages 379-386, XP002452829 ISSN: 0264-6021
- WANG Y ET AL: "Human PAD4 regulates histone arginine methylation levels via demethylimination" SCIENCE, AMERICAN ASSOCIATION FOR THE ADVANCEMENT OF SCIENCE,, US, vol. 306, no. 5694, 8 October 2004 (2004-10-08), pages 279-283, XP002400988 ISSN: 0036-8075
- ANCELIN KATIA ET AL: "Blimp1 associates with Prmt5 and directs histone arginine methylation in mouse germ cells" NATURE CELL BIOLOGY, vol. 8, no. 6, 14 May 2006 (2006-05-14), pages 623-630, XP002452830 ISSN: 1465-7392
- HAYASHI KATSUHIKO ET AL: "Germ cell specification in mice" SCIENCE (WASHINGTON D C), vol. 316, no. 5823, April 2007 (2007-04), pages 394-396, XP002452831 ISSN: 0036-8075

### SEQUENCE LISTING

<110> CAMBRIDGE UNIVERSITY TECHNICAL SERVICES LIMITED
<120> EPIGENETIC REGULATORY COMPLEX FOR CONTROL OF GENE EXPRESSION
<130> P15242WO/DJC
<150> US 60/798,029
   <151> 2006-05-05
<150> GB0608945.2
   <151> 2006-05-05
<160> 11
<170> PatentIn version 3.4
<210> 1
   <211> 19
   <212> DNA
   <213> Homo sapiens
<220>
   <221> misc_feature
   <222> (1)..(19)
   <223> siRNA for PRMT5
<400> 1
   ctcatttgct gacaatgaa 19
<210> 2
   <211> 19
   <212> DNA
   <213> Homo sapiens
<220>
   <221> misc_feature
   <222> (1)..(19)
   <223> siRNA for PRMT5
<400> 2
   ggacctgaga gatgatata 19
<210> 3
   <211> 19
   <212> DNA
   <213> Homo sapiens
<220>
   <221> misc_feature
   <222> (1) .. (19)
   <223> siRNA for PRMT5
<400> 3
   gtttcaagag ggagttcat 19
<210> 4
   <211> 22
   <212> DNA
   <213> Homo sapiens
<400> 4
   ccaggagggg tttcatcaac tg 22
<210> 5
   <211> 24
   <212> DNA
   <213> Homo sapiens
<400> 5
   tgttaccgtc tcacttggtg tttg 24
<210> 6
   <211> 22
   <212> DNA
   <213> Homo sapiens
<400> 6
   acctcacaac tgctgggatt ac 22
<210> 7
   <211> 19
   <212> DNA
   <213> Homo sapiens
<400> 7
   ttcgttttct gcgtccgtg 19
<210> 8
   <211> 24
   <212> DNA
   <213> Homo sapiens
<400> 8
   tttgtcgcag tgtcttatcg taac 24
<210> 9
   <211> 20
   <212> DNA
   <213> Homo sapiens
<400> 9
   taggaaggtg ttggggaggg 20
<210> 10
   <211> 21
   <212> DNA
   <213> Homo sapiens
<400> 10
   atgaggtttg agaagtgtgg c 21
<210> 11
   <211> 21
   <212> DNA
   <213> Homo sapiens
<400> 11
   atcagcggtg gtggtgacag c 21

## Description

### FIELD

The invention is in the field of epigenetic regulation of gene expression. In particular the invention relates to compositions and methods comprising histone methyltransferase activity that are targeted to control gene expression *in vitro.*

### BACKGROUND

Epigenetics concerns the transmission of information from a cell or multicellular organism to its descendants without that information being encoded in the nucleotide sequence of genes. Epigenetic controls are typically established via chemical modification of the DNA or chromatin structure. Gene expression can be moderated, for example, via the methylation and acetylation of histones that are associated with the genomic DNA. Methylation and acetylation of histones tends to occur in the histone tail, a domain that is surface exposed and has a net positive charge due to an abundance of amino acid residues such as arginine (R) and lysine (K). The chemical modification of the histone tail is moderated by enzymes having a histone methyltransferase activity (HMTases) and histone acetyltransferase activity (HATs).

Epigenetic modifications can occur at different times in the normal development of an organism, and also during transformation of normal cells into cancerous cells. Such modifications often result in the silencing or activation of certain genes. In cancer, it is well documented that the majority of tumour cells display abnormal DNA epigenetic imprints (Feinberg AP & Vogelstein B, (1983) Nature 1 (5895):89-92).

Stem cells are cells that are able to both extensively self-renew and differentiate into progenitors. As such, stem cells are also potential candidates for the origin of cancers. Stem cells can have a long lifespan in which they acquire genetic mutations and epigenetic modifications that can increase the tendency toward malignancy. It is postulated that since stem cells occupy a niche that is so finely balanced between the competing interests of proliferation and differentiation, small but profound epigenetic changes can tip the balance towards a cancer stem cell phenotype. An appreciation of why and how epigenetic modifications are regulated is critical to the understanding, detection and treatment of cancer and particularly the treatment of cancer stem cells. Indeed, it is believed that one of the factors present in cases of recurrent and aggressive cancers that are difficult to treat is that the tumours may contain cancer stem cells that do not respond to conventional therapies.

Somatic cell nuclear transfer (SCNT) is used to generate animals for livestock production (for cloning or for stem cell therapy), biomanufacturing of proteins and for disease modelling (Wilmut I, Beaujean N, de Sousa PA, Dinnyes A, King TJ, Paterson LA, Wells DN, Young LE. (2002) Nature. Oct 10;419(6907):583-6). One of the problems associated with the efficiency and success rates obtained for SCNT is that the somatic cell genome comprises extensive and stable epigenetic marks that can hinder successful reprogramming. In addition, the recipient egg cell can comprise factors that exert epigenetic effects that can also contribute to the failure of the procedure. Hence, there is a need to provide compositions and methods to improve the efficiency of SCNT and, thus, the bioprocessing applications that are facilitated by this procedure.

The specification of primordial germ cells (PGCs) in the mouse has provided an attractive experimental model for analysing the effects of epigenetic modifications *in vivo.* A founder population of about 45 PGCs is first detected at E7.5 in mouse embryos (Ginsburg, M., Snow, M. H. & McLaren, A. (1990) Development 110, 521-8). Thereafter, these PGCs migrate and enter the genital ridges from E10.5 onwards, where further extensive epigenetic programming of germ cells continues. By E13.5, germ cells enter into meiotic prophase in the female and mitotic arrest in the male gonad.

Significant epigenetic modifications ensue immediately after the specification of PGCs, including methylation and acetylation of histone tails by HMTases and HATs, respectively (Surani et al., 2004 (CSH Symposium); Seki et al., 2004; Lachner, M., O'Sullivan, R. J. & Jenuwein, T. (2003) J Cell Sci 116, 2117-24, and Vaquero, A., Loyola, A. & Reinberg, D. (2003) Sci Aging Knowledge Environ 2003, RE4). Among the candidate genes postulated to have a role in regulating these epigenetic changes in PGCs are the HMTases that belong to the conserved SET/PR domain protein family. ,

Hence, there is a need to provide reagents and methods for improving the control of epigenetic regulatory mechanisms within the cell. In particular, there is a need for compositions and methods that allow for greater control of gene expression so as to influence cell fate decisions in stem cells and in cancer cells.

### SUMMARY

A first aspect of the invention provides for a polypeptide complex comprising: a first domain having site-specific DNA binding activity, wherein the first domain specifically binds to a B-lymphocyte-induced maturation protein-1 (PRDI/Blimp1)-type consensus binding site, or the first domain comprises the PRDI/Blimp1 polypeptide, or the first domain comprises a DNA binding portion of the PRDI/Blimp1 polypeptide; and a second domain having an arginine methyltransferase activity that provides a symmetrical NG,N'G-dimethylation of an arginine residue and wherein the arginine methyltransferase activity comprises a Protein Arginine Methyltransferase 5 (Prmt5) arginine methyltransferase domain.

The PRDI/Blimp1-type binding site has a consensus sequence of four GGGAAAG motifs two in the in the 5' promoter region of the target gene and two downstream of the transcriptional start site.

A second aspect of the invention provides for a nucleic acid expression vector construct, that is suitable for inducing expression of a polypeptide complex in a mammalian cell, the vector comprising:
one or more coding sequences operably liked to a promoter sequence,
wherein the one or more coding sequences encode at least a first polypeptide domain having site-specific DNA binding activity and at least a second polypeptide domain having an arginine methyltransferase activity, wherein the first domain specifically binds to a PRDI/Blimp1-type consensus binding site, or wherein the first domain comprises the PRDI/Blimp1 polypeptide, or wherein the first domain comprises a DNA binding portion of the PRDI/Blimp1 polypeptide, and the second domain has an arginine methyltransferase activity that provides a symmetrical NG,N'G-dimethylation of an arginine residue located within a polypeptide substrate and wherein the arginine methyltransferase activity is comprised within a Prmt5 arginine methyltransferase domain.

In accordance with a specific embodiment of the invention the polypeptide substrate is a histone. Optionally, the second polypeptide domain is capable of methylating an arginine residue located at position 3 in the tail region of a histone H2A (H2AR3), and also an arginine residue located in the tail region of histone H4.

Suitable expression vectors include plasmids, cosmids, viral vectors and artificial chromosomes such as YACs. Optionally, the promoter sequence can be selected from either a constitutive promoter or an inducible promoter. Suitable inducible promoters include the well-characterised Tet or Tamoxifen regulated systems. Alternative systems may include heat shock sensitive promoters.

The expression vector may comprise an expression cassette in which a first coding sequence encodes the first polypeptide domain and a second coding sequence expresses the second polypeptide domain. The first coding sequence encodes a PRDI/Blimp1 polypeptide and the second coding sequence encodes the Prmt5 polypeptide. In particular embodiments it may be advantageous for the first and second coding sequences to be separated by one or more intervening sequences. Suitably, the one or more intervening sequences can comprise at least one internal ribosome entry sequence (IRES) so as to facilitate bicistronic expression of the first and second coding sequences within the cell. The expression vectors of the invention can also comprise one or more nucleic acid sequences that encode a polypeptide selected from: a selection marker; an antibiotic resistance marker; and a reporter.

A third aspect of the invention provides for an in vitro method for controlling gene expression in a mammalian cell comprising inducing formation within the cell of a polypeptide complex comprising at least a first domain having site-specific PRDI/Blimp1-type consensus binding site DNA binding activity and at least a second domain having a Prmt5 arginine methyltransferase activity. In a specific embodiment, formation of the polypeptide complex is induced within the cell by inducing expression of a PRDI/Blimp1 polypeptide within the cell.

The expression of the PRDI/Blimp1 polypeptide may be induced within the cell by transfection of the cell with an expression vector that encodes a Blimp1 polypeptide, or a derivative or homologue thereof. Optionally, the expression of the PRDI/Blimp1 polypeptide may be induced within the cell by transfection of the cell with the expression vector described previously. Typically the mammalian cell is a human cell derived from tissue or in the form of a cell line. In a particular embodiment of the invention the mammalian cell is a neoplastic or cancerous cell or cell line.

The control of gene expression may result in the control of expression of one of more of the genes selected from the group consisting of *c-Myc; Dhx38; Pcdh7; Q8C9T7; Xylt1; DnaH1; Baip2; Nek7; Dusp2; ENSMUSG00000027041; Sirt4;* and *Blimp1.* The induction of the polypeptide complex in the cell can lead to a reduction in expression of one or more of these genes.

A fourth aspect of the invention provides an in vitro method for promoting self renewal and inhibiting differentiation within a stem cell that has not been derived from a human embryo, comprising inhibiting the formation of a Blimp1/Prmt5 complex within the stem cell. Optionally the stem cell is a mammalian stem cell, suitably a human stem cell. In particular embodiments of the invention the stem cell is selected from the group consisting of an adult stem cell; a stem cell progenitor; and a pluripotent stem cell. It will be appreciated that the invention is in no way directed at reproductive cloning of human or the manipulation and use of human embryos for the purpose of facilitating human reproductive cloning.

In a specific embodiment of this aspect of the invention, inhibition of the formation of a Blimp1/Prmt5 complex within the stem cell is achieved by exposing the cell to a Blimp1 inhibitor compound, a Prmt5 inhibitor compound and/or a Blimp1/Prmt5 complex inhibitor compound. Suitably, the inhibitor compound can be selected from a small molecule inhibitor; a siRNA molecule that binds to Blimp1 or Prmt5 mRNA; an antisense oligonucleotide that binds to Blimp1 or Prmt5 mRNA; and a dominant negative version of the Blimp1 or Prmt5 polypeptide.

Also described is a method of controlling Prmt5 localisation within a cell, typically localisation of endogenous Prmt5, comprising inducing expression within the cell of a Blimp1 polypeptide, thereby inducing formation of a Blimp1/Prmt5 complex within the cell. The Blimp1 induced within the cell can be either exogenous Blimp1 or endogenous Blimp1. Suitably, the cell can be a mammalian stem cell.

A fifth aspect of the invention provides for a method for identifying a Blimp1/Prmt5 complex antagonist molecule comprising determining whether a compound binds to and is capable of moderating the biological activity or function of Blimp1, Prmt5 and/or the Blimp1/Prmt5 complex, and identifying such a binding compound as a Blimp1/Prmt5 complex interacting molecule, characterised in that a compound that binds to and inhibits the biological activity or function of Blimp1, Prmt5 and/or the Blimp1/Prmt5 complex, is identified as an antagonist of the Blimp1/Prmt5 complex.

Also described are uses of the polypeptide complexes of the invention in the treatment of cancer, and to cells - suitably mammalian/human cells - comprising the expression vector constructs described above.

### DESCRIPTION OF THE DRAWINGS

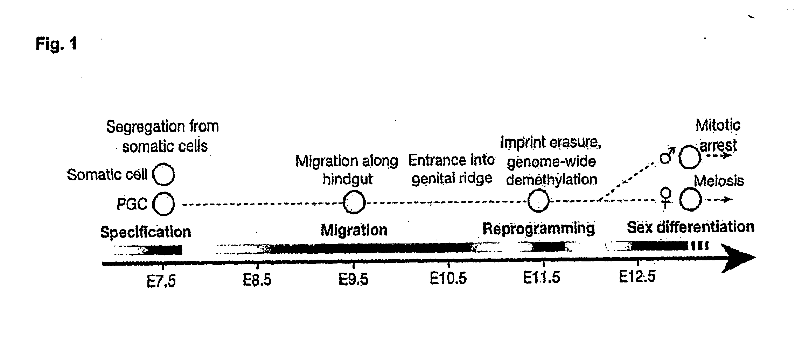

**Figure 1** shows **(a)** a summary of key events during mouse germ cell specification and development from E7.5-E12.5, and **(b)** expression analysis of candidate SET/PR domain genes by PCR of single cell cDNAs from 2 representative founder PGCs (grey) and 2 somatic cells (white). Black fields indicate detection of expression in PGCs and somatic cells;

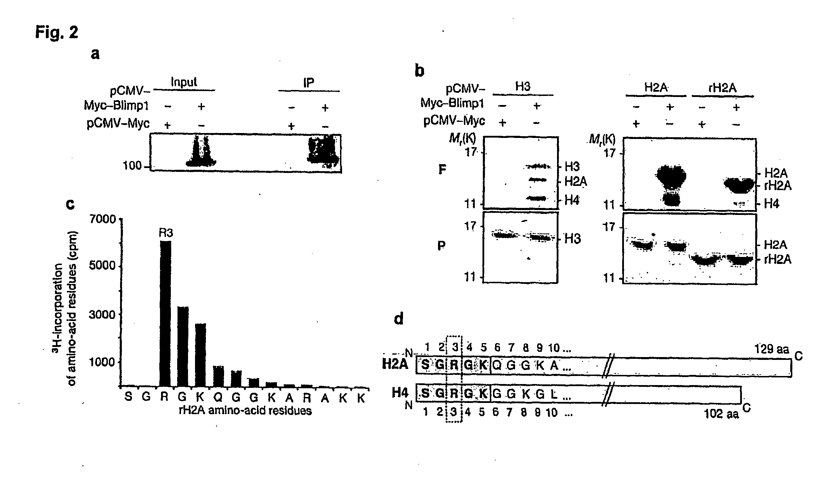

**Figure 2** shows immunoprecipitated mouse Blimp1 complex exhibits an arginine methyltransferase activity, **(a)** Myc-tagged mouse Blimp1 or corresponding control were expressed as indicated in 293T cells. Myc immunoprecipitates were analysed by western blotting using Myc antibodies; **(b)** the same immunoprecipitates were used for HMTase assays against purified histone H3; H2A and recombinant H2A (rH2A). Fluorogramme (F) and Ponceau-stained membrane (P) are shown for each; **(c)** microsequencing of radiolabelled rH2A, the x axis shows amino acids 1 to 14 of rH2A, the y axis indicates the [³H]- incorporation of individual amino acid residues as counts per minute (cpm); and **(d)** Alignment showing the sequence conservation of the extreme N-terminus of H4 and H2A.1;

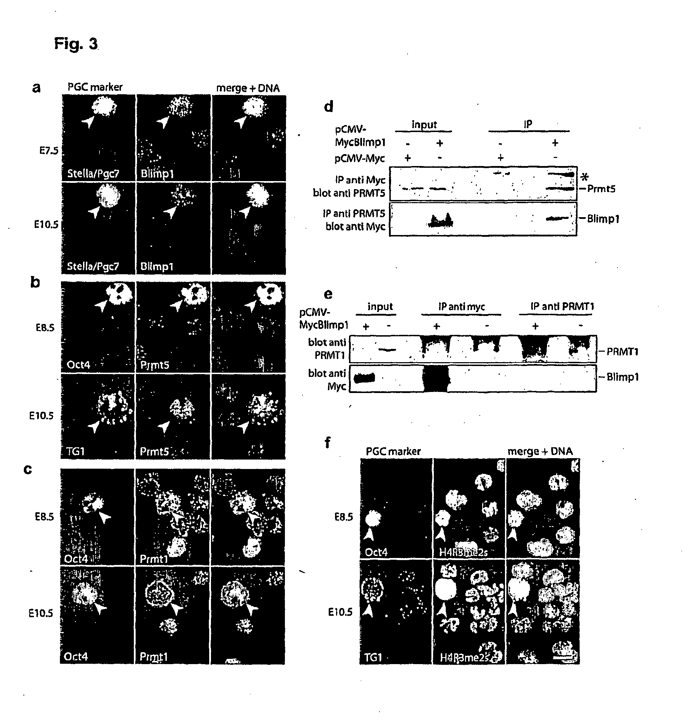

**Figure 3** shows overlapping expression of Blimp1 and Prmt5 in germ cells leads to specific pattern of H2A/H4R3me, **(a,b,c)** Expression pattern of Blimp1, Prmt5 and Prmt1 in PGCs at different stages of development was detected by immunostaining with Blimp1 **(a),** Prmt5 **(b)** and Prmt1 **(c)** specific antibodies, germ cells were detected as shown, using antibodies against stella/PGC7, Oct4 or TG1/SSEA1, merged images are shown with DNA stained with DAPI; **(d,e)** Myc-tagged mouse Blimp1 or corresponding controls were expressed as indicated in 293T cells, Myc, Prmt5 or Prmt1 immunoprecipitates were analysed by Western blotting using Prmt5, Myc or Prmt1 antibodies, the asterisk marks a non-specific signal; and **(f)** H2A/H4 R3 methylation in germ cells was assessed by immunostaining in PGCs at different indicated developmental stages with H4R3me2s antibodies, germ cells are co-stained with antibodies against stage specific markers, namely Oct4 or TG1/SSEA1, merged images are shown with DNA stained with DAPI, scale bar: 10 µm (scale is identical for each picture).

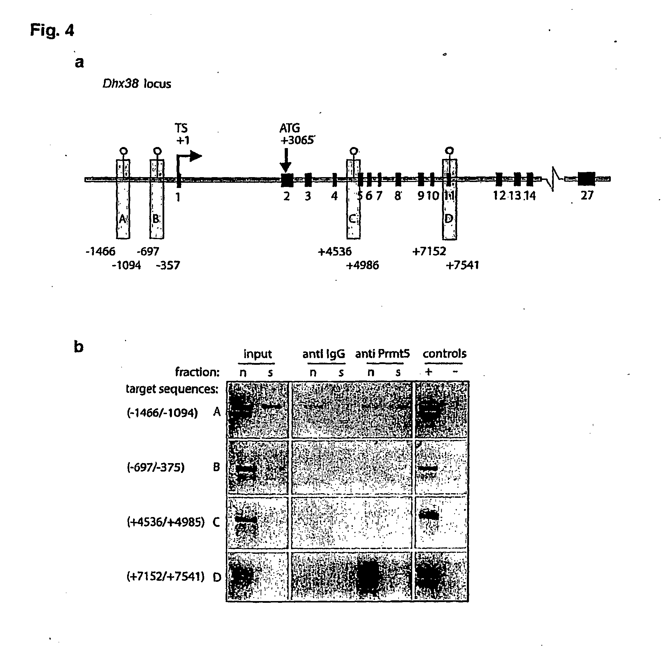

**Figure 4** shows *in vivo* identification of a Blimp1/Prmt5 binding elements within the genomic locus of *Dhx38,* **(a)** positions of putative Blimp1 binding sites in the vicinity of *Dhx38* transcription start (TS) and start codon (ATG), amplified sequences for the ChIP assay are also shown (A, B, C, D), **(b)** Interaction of endogenous Prmt5 with genomic DNA of the *Dhx38* locus by ChIP assay, supernatant (s) or nuclear (n) fractions of cell extracts from isolated genital ridge cells from E10.5 embryos were immunoprecipitated with either Prmt5 or IgG antibodies, tail genomic DNA (+) and water (-) were used as controls.

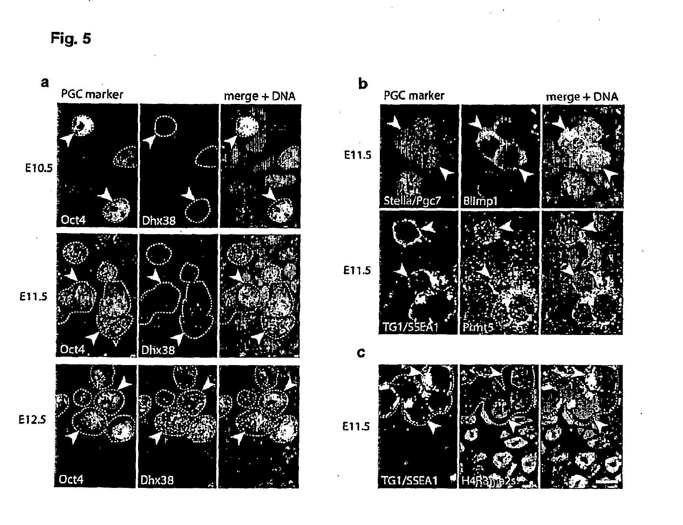

**Figure 5** shows Dhx38 expression is up-regulated in germ cells upon translocation of Blimp1 and Prmt5 from the nucleus to the cytoplasm, resulting in a decrease in the levels of H2A/H4R3me2s modification, immunostaining of (a) Dhx38, (b) Blimp1 and Prmt5, and (c) H2A/H4R3me2s, were performed on cryosections of genital ridges at the developmental stages as indicated, germ cells were detected using specific antibodies; stella/Pgc7, Oct4 or TG1/SSEA1, merged images are shown with DNA stained with DAPI, scale bar: 10 µm (scale is identical for each picture).

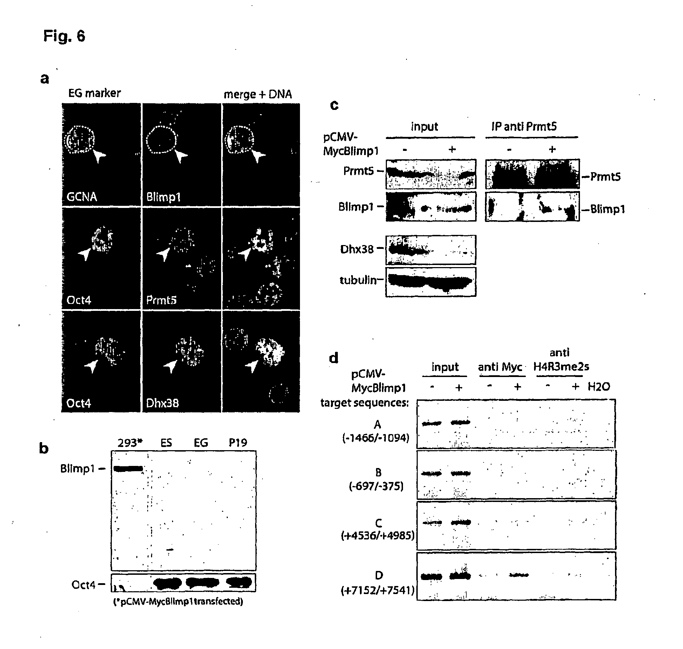

**Figure 6** shows analysis of Blimp1, Prmt5 and Dhx38 in pluripotent EG and embryonic carcinoma (EC) cells (a) Immunostaining for Blimp1, Prmt5 and Dhx38 was performed on EG cells, merged images are shown with DNA stained with DAPI, note the inverse relationship between the expression of *Dhx38* and *Blimp1;* **(b)** Western blot analysis ES, EG or EC (P19) extracts for Blimp1 and Oct4; **(c)** Myc-tagged mouse Blimp1 was expressed as indicated in P19 pluripotent EC cells, Prmt5 immunoprecipitates were analysed by Western blotting using Prmt5, Blimp1 or Dhx38 antibodies, tubulin levels showing equal input lane loading were detected using anti tubulin antibody, note repression of Dhx38 when Blimp1 is introduced in EC (P19) cells; **(d)** Increased levels of H4R3me2s on the *Dhx38* locus in Myc-Blimp1 transfected EC (P19) cells assayed by CHIP, cell extracts from P19 cells were immunoprecipitated with either Myc or H4R3me2s antibodies, A, B, C, D refers to regions in the Dhx38 locus containing Blimp1 binding sites as explained in Figure 4.

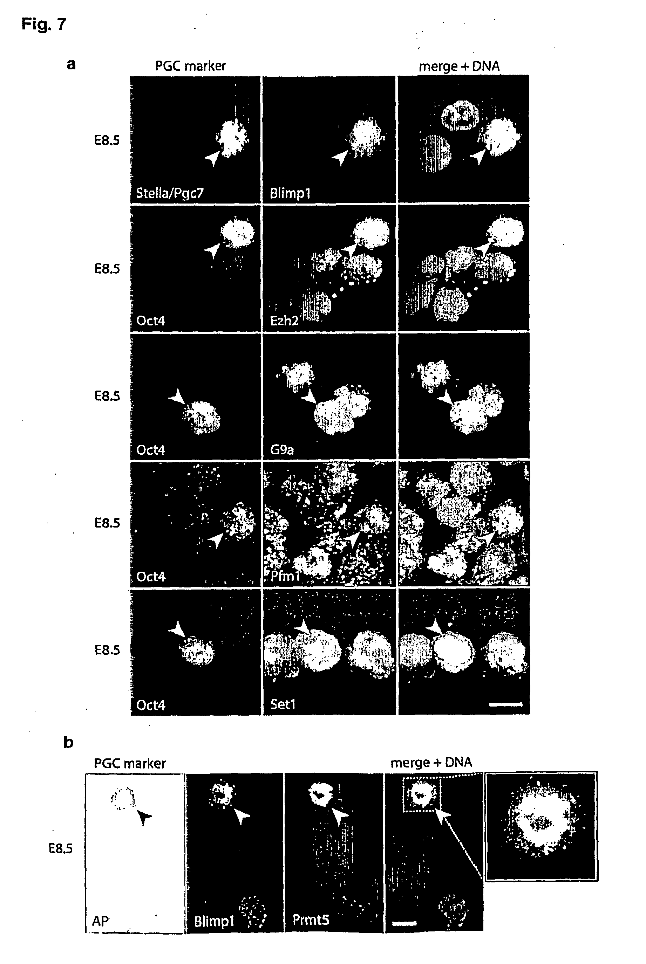

**Figure 7** shows **(a)** immunofluorescence analysis of candidate SET/PR domain genes from the PCR expression screen at E7.5 shown in Figure 1; immunostaining of isolated cells from E8.5 embryos with specific antibodies against specific Histone Methyl Transferases as indicated, germ cells were detected using germ cell-specific antibodies, Oct4 or Stella/PGC7; **(b)** co-immunostaining of Blimp1 and Prmt5 using corresponding antibodies in E8.5 PGCs, germ cells are marked by the expression of tissue non-specific alkaline phosphatase (AP), merged images are shown with DNA stained with DAPI, scale bar: 10 µm (scale is identical for each picture).

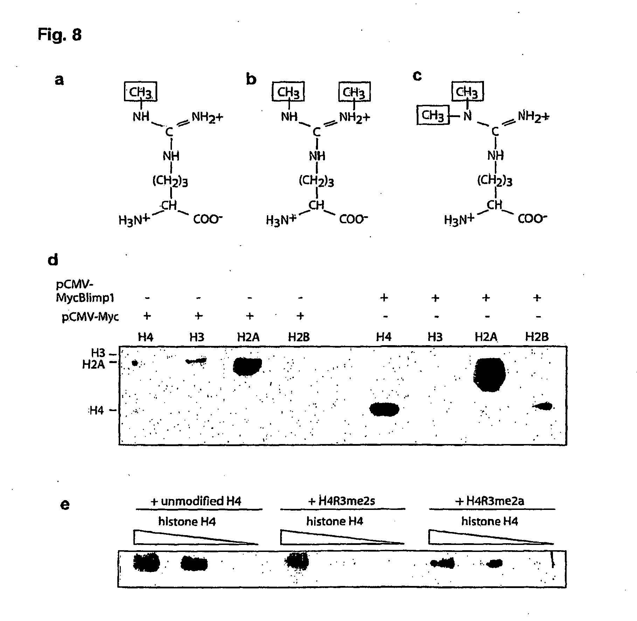

**Figure 8** shows characterisation of H4 R3me2s antibodies, arginine can be modified with a single methyl group (a), or with two methyl groups that can be arranged symmetrically (b) or asymmetrically (c); the antibody against H4 R3me2s (Abcam™) was originally generated using an H4 synthetic peptide with R3 symmetrical demethylation, to test its specificity, Western blot analysis was carried out; (d) against calf thymus histones (H4, H3, H2A, H2B) incubated with and without immunoprecipitated Myc-Blimp1, and (e) after a competition assay against H4 peptides including unmodified, R3me2s and R3me2a, the antibody strongly recognises the symmetrically dimethylated peptide.

### DETAILED DESCRIPTION

Prior to setting forth the detailed description of the invention, a number of definitions are provided that will assist in the understanding of the invention. Unless otherwise defined, all technical and scientific terms used herein have the same meaning as commonly understood by one of ordinary skill in the art to which this invention belongs.

The term 'reprogramming' as used herein, refers to the step of altering or removing epigenetic modifications from the nucleus, of a cell. Reprogramming facilitates a reduction in cell fate commitment and, thus, the differentiation state of the cell as a whole and in particular the nucleus. In essence, reprogramming consists of returning a somatic differentiated or committed nucleus to a gene expression, epigenetic, and functional state characteristic of an embryonic, germ, or stem cell. Reprogramming of somatic cell nuclei is a preferred first step in procedures such as SCNT, but is also of interest in other procedures where control of cell differentiation state - i.e. potency - is important.

The term 'cancer' is used herein to denote a tissue or a cell located within a neoplasm or with properties associated with a neoplasm. Neoplasms typically possess characteristics that differentiate them from normal tissue and normal cells. Among such characteristics are included, but not limited to: a degree of anaplasia, changes in cell morphology, irregularity of shape, reduced cell adhesiveness, the ability to metastasise, increased levels of angiogenesis, increased cell invasiveness, reduced levels of cellular apoptosis and generally increased cell malignancy. Terms pertaining to and often synonymous with 'cancer' include sarcoma, carcinoma, tumour, epithelioma, leukaemia, lymphoma, polyp, transformation, neoplasm and the like.

The term 'epigenetic modification' refers to the chemical marking of the genome. Epigenetic marks can include DNA methylation (imprints) as well as methylation and acetylation of proteins associated with DNA, such as histones. Parent-of-origin-specific gene expression (either from the maternal or paternal chromosome) is often observed in mammals and is due to epigenetic modifications. In the parental germlines, epigenetic modification can lead to stable gene silencing or activation.

'Bioprocessing' refers to techniques in which living cells, or their components are used to produce a desired end product. In the context of the present invention, epigenetic modifications to cells can be used to enhance these cells ability to be used in bioprocessing. Typically bioprocessing techniques include SCNT.

The terms 'derivative or homologues' of DNA binding domains and/or arginine methyltransferases as used herein refer to mRNA and polypeptides that have substantially similar sequence identity to the molecules of the invention respectively. Derivatives and homologues are considered to include orthologues of the sequences from other species and mutants that nonetheless exhibit a high level of functional equivalence. By substantially similar sequence identity, it is meant that the level of sequence similarity is from about 50%, 60%, 70%, 80%, 90%, 95% to about 99% identity. Percent sequence identity can be determined using conventional methods (Henikoff and Henikoff Proc. Natl. Acad. Sci. USA 1992; 89:10915, and Altschul et al. Nucleic Acids Res. 1997; 25:3389-3402). Alternatively, homologues of the polypeptides of the invention can be those sequences that are able to demonstrate the ability to hybridise with the sequences described herein, under conditions of high, medium or low stringency.

The term 'expression vector' is used to denote a DNA molecule that is either linear or circular, into which another DNA sequence fragment of appropriate size can be integrated. Such DNA fragment(s) can include additional segments that provide for transcription of a gene encoded by the DNA sequence fragment. The additional segments can include and are not limited to: promoters, transcription terminators, enhancers, internal ribosome entry sites, untranslated regions, polyadenylation signals, selectable markers, origins of replication and such like. Expression vectors are often derived from plasmids, cosmids, viral vectors and yeast artificial chromosomes; vectors are often recombinant molecules containing DNA sequences from several sources.

The term 'operably linked', when applied to DNA sequences, for example in an expression vector, indicates that the sequences are arranged so that they function cooperatively in order to achieve their intended purposes, i.e. a promoter sequence allows for initiation of transcription that proceeds through a linked coding sequence as far as the termination signal.

A 'polynucleotide' is a single. or double stranded covalently-linked sequence of nucleotides in which the 3' and 5' ends on each nucleotide are joined by phosphodiester bonds. The polynucleotide may be made up of deoxyribonucleotide bases or ribonucleotide bases. Polynucleotides include DNA and RNA, and may be manufactured synthetically *in vitro* or isolated from natural sources. Sizes of polynucleotides are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called "oligonucleotides".

The term 'promoter' as used herein denotes a region within a gene to which transcription factors and/or RNA polymerase can bind so as to control expression of an associated coding sequence. Promoters are commonly, but not always, located in the 5' non-coding regions of genes, upstream of the translation initiation codon. The promoter region of a gene may comprise one or more consensus sequences that act as recognisable binding sites for sequence specific DNA binding domains of DNA binding proteins. Nevertheless, such binding sites may also be located in regions outside of the promoter, for example in enhancer regions located in introns or downstream of the coding sequence.

The term 'isolated', when applied to a polypeptide or complex of polypeptides, is a polypeptide that has been removed from its natural organism of origin. It is preferred that the isolated polypeptide is substantially free of other polypeptides native to the proteome of the originating organism. It is most preferred that the isolated polypeptide be in a form that is at least 95% pure, more preferably greater than 99% pure. In the present context, the term 'isolated' is intended to include the same polypeptide in alternative physical forms whether it is in the native form, denatured form, dimeric/multimeric, glycosylated, crystallised, or in derivatized forms. Reference to a 'complex' as used herein includes instances where the first and second polypeptide domains are comprised within a single polypeptide chain, also where the first and second domains are included within separate polypeptide chains that are non-covalently associated with each other, as well as where post translational covalent bonds are formed to link separate domains together into an associated functional unit.

In a embodiment of the present invention, a novel complex between Blimp1 and Prmt5 is provided that is able to regulate gene expression in mammalian cells via an epigenetic control mechanism.

Somatic cells typically develop along a differentiation pathway progressing from a less specialised to a more specialised or committed state. Less specialised somatic cells can demonstrate the ability to act as progenitor stem cells giving rise to several different cell types. The amount of these different cell types that a given stem cell can act as a progenitor for is typically referred to as the 'potency' of that stem cell. Hence, pluripotent stem cells can act as progenitors for very many different differentiated cell types. If a cell can differentiate into all cells in the body, it is totipotent. If it can differentiate into most cell types, it is pluripotent. Embryonic stem cells are usually referred to as pluripotent as they can generate most cell types in mammals with the exception of extra-embryonic tissues (i.e. trophectoderm). The present invention provides a means for controlling cell fate decisions at the gene expression control level. The protein complex of the invention when expressed or otherwise introduced into mammalian cells biases cell fate decisions away from pluripotency. Conversely, inhibition of activity of the protein complex of the invention in stem cells, or even only of the Blimp1 component, can bias cell fate decisions towards pluripotency and self-renewal.

Another related area of utility for the present invention is in cancer therapy. Most if not all cancers undergo epigenetic changes, including significantly the down-regulation and silencing of tumour suppressor genes and the up-regulation of oncogenes. Reactivation of tumour suppressor genes can ameliorate cancer phenotype as can down-regulation of oncogenes. Hence, a method of controlling gene expression and cell fate decisions *in vivo* is a very promising avenue to cancer therapy.

### B-lymphocyte-induced maturation protein (Blimp1)

Blimp1 is a 100 kDa protein which contains five DNA-binding zinc finger motifs (GENBANK accession no: NM_007548). The human homologue of Blimp1 is referred to as either PRDI-BF1 or PRDM1 (GENBANK accession no: NM_000198). Blimp1 cDNA was originally isolated in a subtractive screen of a B-cell lymphoma cell line (BCL₁) following treatment with cytokines IL-2 and IL-5. Ectopic expression of Blimp1 is sufficient to cause terminal differentiation of BCL₁ cells. Blimp1 is considered to be a 'master regulator' of terminal B-cell development (Yu J. et al. (2000) Mol. Cell. Biol. 20(7): 2592-2603).

In humans, PRDI-BF1, the human orthologue of murine Blimp1, is able to form a complex with the H3 lysine methyltransferase G9a. This complex has been shown to be able to silence the human interferon- β (IFN- β) gene via a chromatin-mediated mechanism in the promoter region of the gene (Gyory I. et al. (2004) Nat. Imm. 5: 299-308).

Blimp1 has been shown to form a complex with factors that are involved in epigenetic modifications. A complex comprising Blimp1 and a histone deacetylase (HDAC) is thought to alter nucleosome structure and inhibit transcription of genes via deacetylation of lysine residues in histone tails. As acetylation of lysine residues effectively neutralises their positive charge, deacetylation restores the charge and leads to modification of nucleosome structure due to stearic and other effects.

Known targets of Blimp1 include c-Myc, IFN-β, CD23, CD22, MHC class II, BSAP- (Pax 5), early B-cell factor and CIITA. All of these genes are subject to transcriptional repression by Blimp1. Transcription of the c-*Myc* gene is repressed by Blimp1 during B-cell differentiation and is an important target of Blimp1 in BCL₁ lymphoma cells. Multiple regions of the Blimp1 molecule, including the N-terminal acidic domain and the region between aa 90 and 464, are required for Blimp1 to repress the c-*Myc* promoter (Yu J. et al., supra).

The c-Myc oncoprotein is critical for regulating growth control, apoptosis, and/or differentiation, and its dysregulation also plays a causal role in a wide variety of neoplasms. Dysregulation of c-Myc expression in B-cells is often tumorigenic. Chromosomal translocations of the c-Myc gene to Ig gene loci are present in most human Burkitt's lymphomas and murine plasmacytomas (Lin K.I. et al. (2000) Mol. Cell Biol. (20)23: 8684-8695).

Murine ES cells can be maintained as a pluripotent, self-renewing population by LIF/STAT3-dependent signalling. STAT3 has been shown to regulate the expression of the Myc transcription factor. Adult stem cells are also believed to require Myc activation. RT-PCR analysis has demonstrated that Myc transcription is elevated in ES cells. Typically ES cells require culture media that includes LIF, otherwise the ES cells tend towards differentiation rather than self renewal. It has been seen that Myc levels in ES cells rapidly collapse following withdrawal of LIF from the culture conditions, indicating that this may be a requirement for ES cell differentiation. Experiments suggest that Myc alone can maintain the ES cell state (i.e. a pluripotent phenotype) at a level comparable with LIF and when Myc is inactivated, the stem cell pool declines (Cartwright P. et al (2005) Development 132: 885-896).

### Protein Arginine Methyltransferase 5 (Prmt5)

There are two known types of histone methyltransferases (HMTases) which include either a SET (Suvar3-9, Enhancer of Zeste, Trithorax) domain found primarily in proteins possessing lysine-specific methylase activity, or an arginine-specific methylase catalytic domain found in PRMTs. PRMTs are divided into types I and II: type I PRMTs catalyze monomethylation and asymmetric demethylation of arginine residues, whilst type II PRMTs catalyze the formation of monomethylated and symmetrically dimethylated arginines. Of the six known PRMTs, only PRMT5 (GENBANK Accession Nos: NM_006109 (human); NM_013768 (mouse)) behaves as a type II PRMT that can target histones. Specifically, PRMT5 has been shown to target specific arginine residues in the H3 and H4 N-terminal tails.

PRMT5 can associate with BRG1- and hBRM-based hSWI/SNF chromatin remodelling complexes. In such complexes, PRMT5 has the ability to stimulate cell growth and anchorage-independent growth by methylating the arginine 8 residue of histone H3 (H3R8) and thereby reducing expression of genes such as ST7 and *NM23* that are known to have roles in tumour suppression (Richard S. et al. (2005) Biochem. J. 388: 379-386). PRMT5 has also been implicated in transcriptional repression of *CYCLIN E* and CAD.

### Blimp1/Prmt5 Complex

The present inventors set out to identify a possible activity of the Blimp1 SET/PR domain since no specific histone methyltransferase activity has yet been attributed to Blimp1 itself. The inventors have demonstrated that Blimp1 can form a novel complex with PRMT5, a complex that in vivo persists in the mouse germ cell lineage until PGC entry into the genital ridges, suggesting that Blimp1 has a continuing role in the early mammalian germ cell lineage. Further analysis, described in more detail below, shows that the novel Blimp1/Prmt5 complex imparts a unique epigenetic signature by methylating histones H2A and H4. It is believed that this is the first instance that methylation of the histone H2A tail has been shown as an epigenetic regulatory mechanism. Further, experiments described in more detail below, show that whilst Prmt5 is present in pluripotent EG and ES cells, Blimp1 is not.

Expression of Blimp1 in a pluripotent EC cell line P19 results in the repression of a gene known to be expressed at high levels in pluripotent cells. The experiments suggest that the Blimp1/Prmt5 complex is an important regulator of gene expression and can exert significant effects upon cell fate choice, particularly in respect of pluripotency and differentiation. As such, an embodiment of the present invention provides a mechanism for gene control based upon utilising the biological activities exemplified by the Blimp1/Prmt5 complex in vitro.

Without wishing to be bound by theory, it is believed that within the complex Blimp1 provides the gene targeting function as it possess the five zinc finger DNA binding domains. Prmt5 provides an arginine methyltransferase activity that serves as a HMTase when localised to the DNA, thereby that leading to repression of gene expression. The human orthologue of Blimp1 is known to bind to the PRDI site in the human IFN-β promoter region (Keller A. D. & Maniatis T. (1991) Genes Dev. 5:868-879). As mentioned above, it is also already known that Blimp1 is capable of repressing c-Myc expression. In accordance with the invention a novel subset of genes have been identified that are shown to be specific targets of the Blimp1/Prmt5 complex and whose expression can be regulated by the novel complex. This subset includes genes having diverse functions, but which are considered to play important roles in regulating potency, the cell cycle, differentiation, cell adhesion, epigenetic reprogramming and possibly also tumour suppression.

In accordance with the invention the presence of the Blimp1/Prmt5 repressor complex correlates with high levels of H2A/H4R3me2s in germ cells. However, during differentiation of B cells to plasma cells, Blimp1 directs G9a-dependent H3K9me2. Thus, Blimp1 is capable of direct diverse cell fate decisions and properties by association with different binding partners, and chromatin remodeling may be central to these processes. In addition to the evident importance of Blimp1 during earlier stages of germ cell specification in mice, the present invention demonstrates an additional involvement of Blimp1 in the formation of a unique epigenetic chromatin signature, as seen in germ cells after the specification of PGCs. However, Blimp1/Prmt5 exits from PGCs nuclei and enters the cytoplasm after E10.5 when extensive genome-wide programming is detected in germ cells. An understanding of the relationship between the Blimp1/Prmt5 complex, the symmetrical dimethylation of arginine 3 on H2A/H4 and the subsequent genome-wide epigenetic reprogramming in germ cells thus provides an insight into the mechanism underlying this critical process and on how nuclear reprogramming is controlled within the cell.

The invention also provides insight into the role of Blimp1/Prmt5 in pluripotent embryonic gonadal (EG) cells whose derivation from PGCs is evidently associated with the loss of Blimp1. The present invention provides direct evidence that Blimp1 is crucial for repressing nascent and migrating PGCs from acquiring an overtly pluripotent stem cell like phenotype. Hence, agonists and antagonists of Blimp1/Prmt5 activity can play an important role in moderating cell fate decisions either away from or toward a pluripotent phenotype respectively. Further, Blimp1 expression within cells provides a mechanism for sequestering Prmt5 arginine methyltransferase activity within the nucleus and away from potential cytoplasmic substrates. Such and effect can be desirable during stem cell differentiation, and general cell cycle progression in the desired cell or cell line.

Particular small nucleic acid molecules that are of use in the invention as inhibitors of Blimp1 and/or Prmt5 are short stretches of double stranded RNA that are known as short interfering RNAs (siRNAs). These interfering RNA (RNAi) techniques allow for the selective inactivation of gene function in vivo. In the present invention, RNAi can be used to knock-down Blimp1 and/or Prmt5 expression in cells. In this process, double stranded mRNAs are recognized and cleaved by the dicer RNase resulting in 21-23 nucleotide long stretches of RNAi. These RNAis are incorporated into and unwound by the RNA-inducing silencing complex (RISC). The single antisense strand then guides the RISC to mRNA containing the complementary sequence resulting in endonucleolytic cleavage of the mRNA (Elbashir et al. (2001) Nature 411; 494-498). Hence, this technique provides a means for the targeting and degradation of Blimp1 and/or Prmt5 mRNA in somatic cells destined for use in bioprocessing applications or for when promotion of a self-renewing pluripotent phenotype is desirable. Techniques for generating siRNA for Prmt5 have been described in the art (Richard S., *supra*). Examples of suitable siRNA sequences for targeting human PRMT5 include:
5' CTCATTTGCTGACAATGAA 3' [SEQ ID NO: 1]
5' GGACCTGAGAGATGATATA 3' [SEQ ID NO: 2]
5' GTTTCAAGAGGGAGTTCAT 3' [SEQ ID NO: 3]

The Blimp1/Prmt5 complex of the present invention can be used to identify other proteins and polypeptides that interact with it in the cellular environment. Conventional techniques for determining protein-protein interactions, such as the yeast two-hybrid screen, can be used to identify potential agonists and antagonists of Blimp1/Prmt5 complex interactivity. It is also within the remit of the invention to identify small molecules that inhibit the association of Blimp1 with Prmt5, for example, by masking or disrupting interaction with those domains of Blimp1 that are known to directly interact with other proteins (Yu J. et al. supra). Blimp1, Prmt5 and/or Blimp1/Prmt5 complex protein-protein interactions or protein-small molecule interactions can be investigated using technologies such as a BlAcore^{®} which detects molecular interactions using surface plasmon resonance (BlAcore, Inc., Piscataway, NJ; see also www.biacore.com).

Screening of molecules and proteins for binding to the Blimp1/Prmt5 complex can be performed via automated high-throughput screening procedures. Hence, the invention provides methods for identifying Blimp1/Prmt5 complex interacting molecules via detection of a positive binding interaction between the Blimp1/Prmt5 complex and a target molecule. Further screening steps may be used to determine whether the identified positive binding interaction is of pharmacological importance - i.e. whether the target molecule is capable of moderating Blimp1, Prmt5 and/or Biimp1/Prmt5 complex biological activity or function. If a molecule with a positive moderating effect is identified, the molecule is classified as a 'hit' and can then be assessed as a potential candidate drug. Additional factors may be taken into consideration at this time or before, such as the absorption, distribution, metabolism and excretion (ADME), bio-availability and toxicity profiles of the molecule, for example. If the potential drug molecule satisfies the pharmacological requirements it is deemed to be pharmaceutically compatible. Suitable compositions can be formulated for testing the activity in-vitro and in-vivo in accordance with standard procedures known in the art.

### EXAMPLES

### Methods and reagents

*Embryo isolation.* Primordial germ cells were isolated from embryos at different stages of development from outbred MF1 mice. The day of the vaginal plug was designated as E0.5. Single cell cDNA libraries originated from previously published work (Saitou, M., Barton, S. C. & Surani, M. A. (2002) Nature 418, 293-300).

*Immunostaining.* Dissected embryonic pieces containing germ cells were treated with trypsin to prepare a single cell suspension. The cells were subsequently allowed to settle on poly-L-lysine coated slides, fixed with 2%PFA and washed three times with PBS and processed further. Whole embryonic genital ridges at E11.5 were dissected, washed in PBS, fixed for two hours at 4°C in 4%PFA, washed in PBS and allowed to settle in 20% sucrose overnight at 4°C. They were embedded in O.C.T (BDH) and cryosectioned. Single cells or sections were permeabilised in IF buffer (PBS ; 0,1% triton ; 10mg/ml BSA). Primary antibody incubation was carried out overnight at 4°C, followed by three washes in IF buffer and incubation with secondary antibodies (Alexa 564, Alexa 488 ; Molecular Probes) for two hours at room temperature, and washed in PBS. Slides were then mounted in vectashields with DAPI (Vector laboratories). Immunofluorescence was visualised on a BioRad radiance 2000 confocal microscope. The following antibodies and dilutions were used: PGC7 (from T.Nakano; 1:2500), Oct4 (BD Transduction Laboratories ; 1:200), TG1 (mouse germ cell specific anti-SSEA1 monoclonal antibody; 1 :1), Blimp1 (from K.Calame ; 1:10), Prmt5 (Upstate; 1:250), Prmt1 (Upstate, 1:200), H4R3me2s (Abcam ; 1:1000), Dhx38/Prp16 (Proteintech Group ; 1 :200), Ezh2 (Upstate ; 1 :50), G9a (Abcam™ ; 1 :100), Pfm1 (Abcam™ ; 1 :50), Set1 (from W.Herr; 1 :200).

*Immunoprecipitation assays and preparation of nuclear extracts.* The coding region of mouse Blimp1 was amplified by RT-PCR and the product cloned into pcDNA3-MycHisA resulting in the construct pCMV-MycBlimp1. 293T or P19 cells, transfected with either the original vector (called pCMV-Myc), or with pCMV-MycBlimp1 were washed with PBS and lysed in IP buffer containing 150mM NaCl, 1% NP40, 0,1% triton, 50mM Tris pH 8.0 and a complete protease inhibitor cocktail (Roche). In a typical Immunoprecipitation reaction 2x10⁷ cells were used. Whole cell extracts were incubated with 2 µg of Myc antibodies (New England Biolabs). Alternatively, either Prmt5 or Prmt1 antibodies were used (Upstate) overnight at 4°C. 30 µl of protein A/G sepharose beads were then added at 4°C for 2 hours. The beads were washed five times in I P buffer. Bound proteins were eluted by boiling in Laemli sample buffer. Nuclear extracts for ES, EG or P19 cells were prepared according to manufacturer instruction (nuclear extract kit; Active Motif), and 25µg of nuclear fraction was used per loading.

*In vitro methyltransferase assays.* Beads from immunoprecipitation assays from 293T cells transfected with either pCMV-Myc or pCMV-MycBlimp1, were processed for two additional washes in HMTase buffer (25mM NaCl, 25mM Tris pH 8.8) and used in HMTase assay as described¹⁶ with slight modifications. Briefly, 1 µg of H3 or H2A (Roche) or recombinant H2A (kind gift from A. Brehm) as substrate and 2µCi of S adenosyl-L [methyl-³H] methionine ([³H]SAM; Amersham Biosciences) as the methyl donor, were incubated in a mixture of 20µl of HMTase buffer for three hours at 37°C. Proteins were resolved in an 18% SDS-Page gel, transferred on a PDVF membrane and visualized by Ponceau staining and fluorography. *In vitro* methylated rH2A was microsequenced by sequential Edman degradation (Protein and Nucleic acid Chemistry Facility, Cambridge University, UK), followed by determination of ³H incorporation of individual amino acids by scintillation counting. To examine H2A/H4 specific methylation, H4, H3, H2A and H2B (Roche) were incubated in the presence of immunoprecipitated Blimp1 complex and SAM as described above and western blot analysis was performed using H4R3me2s antibodies (Abcam™, Cambridge, UK) that were tested for their specificity by competition assay (see supplementary data Fig.8).

*Chromatin Immunoprecipitation (CHIP) cloning and ChIP.* Single cell suspensions were crosslinked in 1% formaldehyde for 10min at room temperature. The cells were subsequently disrupted and nuclei were lysed in 50mM Tris pH8.0, 10mM EDTA, 1%SDS followed by sonication. The extract was subjected to immunoprecipitation as described above, with the addition of purified IgG (Santa Cruz) as negative control. Beads were then eluted twice in 50mM NaHCO3, 1 % SDS and the supernatants were treated with proteinase K for 5hr at 65°C, followed by phenol/chloroform purification and ethanol precipitation. PRMT5 (Upstate) or H4R3m32 (Abcam™) ChiP samples were then analysed by standard PCR reaction using the following primers (1for ccaggaggggtttcatcaactg [SEQ ID NO: 4] and 1rev tgttaccgtctcacttggtgtttg [SEQ ID NO: 5]; 2for acctcacaactgctgggattac [SEQ ID NO: 6] and 2rev ttcgttttctgcgtccgtg [SEQ ID NO: 7]; 3for tttgtcgcagtgtcttatcgtaac [SEQ ID NO: 8] and 3rev taggaaggtgttggggaggg [SEQ ID NO: 9]; 4for atgaggtttgagaagtgtggc [SEQ ID NO: 10] and 4rev atcagcggtggtggtgacagc [SEQ ID NO: 11]). In the case of the CHIP cloning assay, two successive rounds of immunoprecipitation using anti Myc antibodies were performed. This was followed by precipitation of DNA that was blunted by T4 DNA polymerase and then ligated to annealed JW102 and JW103 ²⁵. Ligation products were then PCR amplified with JW102 (one cycle 55°C 2min ; 72°C 5min; 94°C 2min and 20 cycles 94°C 30s ; 55°C 30s ; 72°C 1 min and finally one cycle 72°C 5min). PCR products were cloned into pGEM-T (Promega), colonies were checked for inserts by PCR and the products sequenced by standard methods. Blast searches were subsequently done using an online bioinformatics resource such as Ensembl (http://www.ensembl.org).

### Example 1: Identification of Blimp1 activity

Significant epigenetic modifications ensue immediately after the specification of mouse PGCs, including methylation and acetylation of histone tails by methyltransferases (HMTases) and acetyltransferases (HATs), respectively. Among the candidate genes that may have role in regulating these epigenetic changes in PGCs are the HMTases that belong to the conserved SET/PR domain protein family. Twenty-five candidate SET/PR domain containing genes were analyzed for expression in E7.5 PGCs and surrounding somatic cells and found that *Blimp1, G9a*, *Set1, Ezh2* and *Pfm1* are expressed in the embryonic region containing PGCs (Fig.1b and Fig.7). However, only *Blimp1* expression was restricted to PGCs at E7.5, and its expression persisted thereafter in germ cells.

To investigate the activity of the Blimp1 SET/PR domain, it was first established that a Myc-tagged mouse Blimp1 when transiently expressed in 293T cells could be efficiently immunoprecipitated using Myc antibodies (Fig.2a). The immunoprecipitate was then subjected to a standard radioactive histone methyltransferase assay on histone H3 (Rea, S. et al. (2000) Nature 406, 593-9). A comparatively weak signal corresponding to H3 was observed, but surprisingly, significant bands were also detected corresponding to histones H2A and H4 (Fig.2b), the latter being present as low level contaminants in the H3 preparation as determined by Western blotting (data not shown). It was reasoned that the weak signal on H3 in vitro could be due to Blimp1-associated G9a as reported previously in B cells (Gyory et al, *supra*). However, germ cells do not exhibit significant histone H3 Lysine 9 dimethylation (H3K9me2), which is the primary modification attributed to G9a, nor is PGC specification detectably affected by loss of G9a function. It was therefore decided to focus on the observed activity of immunoprecipitated Blimp1 on H2A and H4 histones.

Due to the novelty of the discovery of the H2A tail methylation, it was decided to first test the methyltransferase activity of the immunoprecipitate on calf thymus and recombinant H2A preparations, and found that it has a strong methylation activity for this histone (Fig.2b). To identify the target amino acid residue(s) on the H2A tail, the radiolabelled recombinant protein product of the methyltransferase assay was subjected to sequential Edman degradation. Scintillation counting of the released amino acid fraction detected radiolabelling of rH2A R3 (Fig.2c). The same results were obtained with calf thymus and recombinant H4 preparations (data not shown). This is a consistent finding when considering the amino acid sequence conservation between the first N-terminal residues of histones H2A and H4 (Fig.2d). H4 R3 methylation is known to play an important role in transcriptional regulation. However, these results predict the existence of an additional novel methylation of R3 on histone H2A.

### Example 2: Identification of a Blimp1/Prmt5 complex

As SET/PR domains are associated with histone methyltransferase activity solely on lysine residues, it was reasoned that the arginine methylation activity detected above could not be due to Blimp1, and must implicate another HMTase present in the immunoprecipitate. Two protein arginine methyltransferases, Prmt1 and Prmt5, have previously been reported to mediate histone H4 R3 methylation. Prmt1 is a class I arginine methyltransferase leading to NG-monomethylarginine (Rme1) and asymmetrical NG,N'G-dimethylarginine (Rme2a) on diverse types of substrates. As mentioned above, Prmt5 belongs to class II arginine methyltransferase, which is responsible for monomethylation of arginine (Rme1) and symmetrical NG,N'G-dimethylation (Rme2s) (Fig.8) 20. To test if either of these proteins could be associated with Blimp1 in vivo, their expression was analysed in nascent PGCs and surrounding somatic cells using single cell cDNA libraries as described above. It was found that Prmt1 was excluded from PGCs at E7.5, while Prmt5 was present in both PGCs and somatic cells (data not shown). At the protein level however, Prmt5 showed nuclear staining and became highly enriched in PGCs compared to somatic cells from E8.5 onwards (Fig3a, b; Fig.S1b and see below). Prmt1, on the other hand, was detected mainly in the cytoplasm of germ cells at these stages (Fig.3c).

Next, it was decided to investigate whether it is Prmt5 or Prmt1 that interacts with Blimp1. Indeed, it was found that Myc-tagged Blimp1 could efficiently co-immunoprecipitate endogenous Prmt5 in 293T cells (Fig.3d). Conversely, Prmt5 could also pull down Myc-tagged Blimp1 (Fig.3d). By contrast, detection of any interaction between Myc-tagged Blimp1 and endogenous Prmt1 (Fig.3e) could not be found. These experiments confirmed that Blimp1 and Prmt5 can form a complex in 293T cells. Considering the overlapping expression of these proteins in PGCs (Fig.3a, b; Fig.S1 b), it is reasoned that Blimp1 and Prmt5 are part of the same protein complex in PGCs, and that this complex can occur elsewhere during development or in normal as well as neoplastic adult tissues.

### Example 3: Blimp1/Prmt5 complex activity in vivo

The specificity of the antibody raised against H4R3me2s was tested and showed that it efficiently recognizes both histones H2A and H4 from calf thymus (Fig.8b, left panel, H2A and H4 are also present as contaminants in H3 and H2B preparations). In a competition assay, this antibody was efficiently titrated out by a H4 (1-9C) peptide containing R3me2s (Fig.S2c). Moreover, this antibody also recognised the product of Blimp1/Prmt5 HMTase assay on both H4 and H2A (Fig.S2b, right panel), hence adding further evidence that it is the symmetrical dimethylation activity of Prmt5 and not the asymmetrical dimethylation activity of Prmt1, which is attributed to the complex.

Using the H2/H4R3me2s modification-specific antibody, PGCs isolated from early embryos were analysed by immunocytochemistry. At E8.5, H2A/H4R3me2s was evident in both PGCs and somatic cells (Fig.3f), but at E10.5, much higher accumulation of H2A/H4 R3me2s was predominantly observed in germ cells (Fig.3f). However, when another antibody was used, which recognises mono- and/or asymmetrical di-methylation of H4R3 (H4R3me1 and H4R3me2a), it was observed that this modification in PGCs occurred at E8.5, but not at E10.5 (data not shown). The combined data with these two antibodies shows a progression towards H2A/H4R3me2s in PGCs from E8.5 to E10.5. These results demonstrate the presence of a specific chromatin signature during germ cell development, which is believed to be due to the combined presence of Prmt5 and Blimp1. It is important to note that there are multiple additional histone tail modifications contributing to the germ cell specific chromatin state. It has recently been shown that loss of function of Blimp1 leads to aberrant development of founder PGC-like cells that cease to proliferate (Ohinata, Y. et al. (2005) Nature 5, 5).

### Example 4: Identification of in vivo targets of the Blimp1/Prmt5 complex

As shown previously, Blimp1 can direct gene regulation during cell differentiation via the recruitment of interacting factors to specific sites. To identify putative Blimp1 targets, a chromatin immunoprecipitation cloning approach was adopted, in which a Myc-tagged version of Blimp1 was first over expressed in 293T cells. Nuclear extracts from these 293T cells were then immunoprecipitated with Myc antibodies, and the immunoprecipitated DNA was extracted, purified, blunted by ligated linkers, PCR amplified and cloned. A number of clones were selected and analysed by sequencing, followed by blast analysis to map the cloned inserts. Out of 32 clones, 11 corresponded to regions located in the vicinity or within likely regulatory sequences of known genes (see Table 1).

**Table 1**

| Gene | hit | putative function |
|---|---|---|
| *Dhx38* | 5'upstream region | signalling / cell cycle |
| *Pcdh7* | 5'upstream region | cell adhesion |
| *Q8C9T7* | intron | chromatin regulation |
| *Xylt1* | intron | metabolism |
| *DnaH1* | intron | cytoskeleton / sperm motility |
| *Baip2* | intron | signalling / cytoskeleton organization |
| *Nek7* | 1 st. intron | cell cycle regulation |
| *Dusp2* | 5'upstream region | signalling / cell cycle |
| *ENSMUSG00000027041* | intron | metabolism |
| *Sirt4* | 2nd intron | metabolism / transcriptional regulation |
| *Blimp 1* | 3'UTR | transcription |

### Example 5: Characterisation of Dhx38 gene expression control by the Blimp1/Prmt5 complex

Among the putative Blimp1 targets identified, *Dhx38* was picked for further study. *Dhx38* is a conserved gene encoding a DEAH box containing RNA helicase (also known as Prp16), which in *C.elegans* is essential for post-translational regulation of sex-determining genes during the sperm to oocyte switch (Graham, P. L. & Kimble, J. (1993). Genetics 133, 919-31).

When *Dhx38* locus was examined in detail, it was found that it contains four GGGAAAG motifs, corresponding to the Blimp1 consensus binding site; two in the 5' region and two downstream of the transcriptional start site (Fig.4a). While the currently available Blimp1 antibody works for immunostaining procedures, it cannot be used to efficiently immunoprecipitate Blimp1 (data not shown). Thus Prmt5 was used to address whether *Dhx38* is a target of the Blimp1/Prmt5 complex in germ cells. A ChIP assay using Prmt5 antibodies was performed on PGCs contained within cell suspensions of genital ridges from E10.5 embryos, a stage when Blimp1 and Prmt5 are co-expressed in germ cell nuclei (see above). Indeed, it was found that Prmt5 could specifically pull down a selected sequence spanning nucleotides +7152 to +7541, encompassing exon 11 of the *Dhx38* gene, which contains a Blimp1 consensus-binding site (Fig.4b). The other three putative binding sites were not associated with Prmt5 in our ChIP assay. These results indicate that Prmt5 is recruited to Blimp1 targets such as *Dhx38,* suggesting therefore that the Blimp1/Prmt5 complex regulates expression of such target genes in germ cells.

With the evidence that *Dhx38* is a target of the Blimp1/Prmt5 complex in germ cells, the expression/repression of Dhx38 in PGCs was looked at. It was found that Dhx38 was undetectable at E10.5 and E11.5 (Fig.5a). However, by E12.5, Dhx38 is up regulated in both female and male PGCs (Fig.5a), which, strikingly, occurs concomitantly with the delocalisation of Prmt5 and Blimp1 from the nucleus to the cytoplasm in germ cells at E11.5 (Fig.5b). At E12.5, neither Prmt5 nor Blimp1 are present in PGC nuclei (data not shown). Dhx38 up-regulation immediately precedes meiotic and mitotic arrest of female and male germ cells, respectively. Hence, there is an inverse relationship between the expression of Blimp1/Prmt5 and Dhx38. These results indicate that Blimp1 and Prmt5 can function in the transcriptional repression of target genes, such as *Dhx38,* in the germ line. This repression appears to be released when germ cells have entered the genital ridge and both Blimp1 and Prmt5 have undergone nuclear-to-cytoplasmic translocation. It should be noted that histone arginine methylation has been mainly associated with transcriptional activation, although recently, Prmt5 associated H3R8me activity has been correlated with reduced gene expression (Pal S. et al, *supra*). Furthermore, since both *Blimp1* and *Prmt5* have previously been reported to be transcriptional repressors, it is possible that the symmetrical arginine dimethylation associated with the Blimp1/Prmt5 complex in germ cells described here would favor gene repression.

### Example 6: Blimp1-Prmt5 complex in pluripotent stem cells

To gain additional insight into the role of the Blimp1/Prmt5 complex, it was decided to examine pluripotent embryonic germ (EG) cells. EG cells can be derived from isolated PGCs *in vitro* and are therefore considered to be the closest cell line equivalent to PGCs. It was found that EG cells are also positive for Prmt5, but unlike PGCs, they lack Blimp1 (Fig.6a, b). Consistent with this observation, it was found that EG cells are also positive for Dhx38, suggesting that expression of this gene may be due to the absence of Blimp1 (Fig.6a, b). To restore the Blimp1/Prmt5 repression complex, Myc-tagged Blimp1 was over-expressed in EG cells. However, such attempts lead to strong cytotoxicity after as little as 12h of cell transfection, while cell viability of pCMV-Myc transfected control EG cells was not compromised (data not shown). Similar results were also obtained with pluripotent embryonic stem (ES) cells (data not shown).

A pluripotent mouse embryonic carcinoma (EC) cell line, P19, which also has characteristics of pluripotency similar to EG and ES cells was then chosen. Indeed, P19 cells, like ES/EG cells, show expression of *Prmt5* and *Dhx38,* but not of *Blimp1* (Fig.6b, c). However, it was found that these cells could tolerate expression of *Blimp1,* and therefore immunoprecipitation was used, which indeed confirmed that over-expressed Myc-Blimp1 interacts with endogenous Prmt5 in mouse EC cells (Fig.6c). Notably, this transient Blimp1/Prmt5 complex subsequently caused down regulation of Dhx38 in P19 cells (Fig.6c). ChIP analysis confirmed this down regulation of *Dhx38* to be accompanied by increased levels of H2A/H4R3me2s at the *Dhx38* locus (Fig.6d). These observations support the notion that Blimp1/Prmt5 complex is responsible for the repression of target genes such as *Dhx38,* which is also likely in PGCs.

Although particular embodiments of the invention have been disclosed herein in detail, this has been done by way of example and for the purposes of illustration only. The aforementioned embodiments are not intended to be limiting with respect to the scope of the appended claims, which follow. It is contemplated by the inventors that various substitutions, alterations, and modifications may be made to the invention without departing from the scope of the invention as defined by the claims.

## Claims

1. A polypeptide complex comprising:
a first domain having site-specific DNA binding activity, wherein
the first domain specifically binds to a B-lymphocyte-induced maturation protein-1 (PRDI/Blimp1 )-type consensus binding site; or
the first domain comprises the PRDI/Blimp1 polypeptide; or
the first domain comprises a DNA binding portion of the PRDI/Blimp1 polypeptide; and
a second domain having an arginine methyltransferase activity that provides a symmetrical NG,N'G-dimethylation of an arginine residue and wherein the arginine methyltransferase activity comprises a Protein Arginine Methyltransferase 5 (Prmt5) arginine methyltransferase domain.

2. A nucleic acid expression vector construct, that is suitable for inducing expression of a polypeptide complex in a mammalian cell, the vector comprising:
one or more coding sequences operably linked to a promoter sequence,
wherein the one or more coding sequences encode at least a first polypeptide domain having site-specific DNA binding activity and at least a second polypeptide domain having an arginine methyltransferase activity, wherein
the first domain specifically binds to a PRDI/Blimp1-type consensus binding site; or
the first domain comprises the PRDI/Blimp1 polypeptide; or
the first domain comprises a DNA binding portion of the PRDI/Blimp1 polypeptide, and
the second domain has an arginine methyltransferase activity that provides a symmetrical NG,N'G-dimethylation of an arginine residue located within a polypeptide substrate and wherein the arginine methyltransferase activity is comprised within a Prmt5 arginine methyltransferase domain.

3. The expression vector of claim 2, wherein the polypeptide substrate is a histone.

4. An in vitro method for controlling gene expression in a mammalian cell comprising inducing formation within the cell of a polypeptide complex comprising at least a first domain having site-specific PRDI/Blimp1-type consensus binding site DNA binding activity and at least a second domain having a Prmt5 arginine methyltransferase activity.

5. The method of claim 4, wherein formation of the polypeptide complex is induced within the cell by inducing expression of a PRDI/Blimp1 polypeptide within the cell.

6. The method of claims 4 or 5, wherein the mammalian cell is selected from a human cell; and/or a neoplastic or cancerous cell.

7. An in vitro method for promoting self renewal and inhibiting differentiation within a stem cell that has not been derived from a human embryo, comprising inhibiting the formation of a Blimp1/Prmt5 complex within the stem cell.

8. The method of claim 7, wherein the stem cell is selected from: a mammalian stem cell; a human stem cell; an adult stem cell; a stem cell progenitor; and a pluripotent stem cell.

9. The method of any of claims 7 or 8, wherein inhibition of the formation of a Blimp1/Prmt5 complex within the stem cell is achieved by exposing the cell to a Blimp1 inhibitor compound, a Prmt5 inhibitor compound and/or a Blimp1/Prmt5 complex inhibitor compound.

10. The method of claim 9, wherein the inhibitor compound is selected from the group consisting of a small molecule inhibitor; a siRNA molecule that binds to Blimp1 or Prmt5 mRNA; an antisense oligonucleotide that binds to Blimp1 or Prmt5 mRNA; and a dominant negative version of the Blimp1 or Prmt5 polypeptide.

11. A method for identifying a Blimpl/Prmt5 complex antagonist molecule comprising determining whether a compound binds to and is capable of moderating the biological activity or function of Blimp1, Prmt5 and/or the Blimp1/Prmt5 complex, and identifying such a binding compound as a Blimp1/Prmt5 complex interacting molecule, **characterised in that** a compound that binds to and inhibits the biological activity or function of Blimp1, Prmt5 and/or the Blimp1/Prmt5 complex, is identified as an antagonist of the Blimp1/Prmt5 complex.

## Patentansprüche

1. Polypeptidkomplex umfassend:
eine erste Domäne mit einer ortsspezifischen DNA-Bindungsaktivität, wobei
sich die erste Domäne spezifisch an eine Konsensusbindungsstelle vom Typ des B-Lymphozyten-induzierten Reifungsproteins-1 (PRDI/Blimp-1) bindet, oder
die erste Domäne das PRDI/Blimp-1-Polypeptid umfasst; oder
die erste Domäne einen DNA-Bindungsabschnitt des PRDI/Blimp-1-Polypeptids umfasst; und
eine zweite Domäne mit einer Arginin-Methyltransferaseaktivität, die eine symmetrische NG,N'G-Dimethylierung eines Argininrests bereitstellt und wobei die Arginin-Methyltransferaseaktivität eine Arginin-Methyltransferasedomäne einer Protein-Arginin-Methyltransferase-5 (Prmt5) umfasst.

2. Vektorkonstrukt zur Expression einer Nukleinsäure, welches dazu geeignet ist, die Expression eines Polypeptidkomplexes in einer Säugetierzelle zu induzieren, wobei der Vektor umfasst:
eine oder mehrere Kodierungssequenzen, die funktionsfähig mit einer Promotorsequenz verbunden sind,
wobei die eine oder mehrere Kodierungssequenzen für mindestens eine erste Polypeptiddomäne mit einer ortsspezifischen DNA-Bindungsaktivität und mindestens eine zweite Polypeptiddomäne mit einer Arginin-Methyltransferaseaktivität kodieren, wobei die erste Domäne sich spezifisch an eine Konsensusbindungsstelle vom Typ PRDI/Blimp-1 bindet; oder
die erste Domäne das PRDI/Blimp-1-Polypeptid umfasst; oder die erste Domäne einen DNA-Bindungsabschnitt des PRDI/Blimp-1-Polypeptids umfasst, und
die zweite Domäne eine Arginin-Methyltransferaseaktivität besitzt, die eine symmetrische NG,N'G-Dimethylierung eines Argininrests, der innerhalb eines Polypeptidsubstrats lokalisiert ist, bereitstellt, und wobei die Arginin-Methyltransferaseaktivität innerhalb einer Prmt5-Arginin-Methyltransferasedomäne enthalten ist.

3. Expressionsvektor nach Anspruch 2, wobei das Polypeptidsubstrat ein Histon ist.

4. In-Vitro-Verfahren zur Kontrolle von Genexpression in einer Säugetierzelle, welches die Induktion der Bildung eines Polypeptidkomplexes innerhalb der Zelle umfasst, welcher mindestens eine erste Domäne mit ortsspezifischer DNA-Bindungsaktivität einer Konsensusbindungsstelle vom Typ PRDI/Blimp-1 und mindestens eine zweite Domäne mit einer Prmt5-Arginin-Methyltransferaseaktivität umfasst.

5. Verfahren nach Anspruch 4, wobei die Bildung des Polypeptidkomplexes innerhalb der Zelle durch die Induktion der Expression eines PRDI/Blimp-1-Polypeptids innerhalb der Zelle induziert wird.

6. Verfahren nach Anspruch 4 oder 5, wobei die Säugetierzelle ausgewählt ist aus einer humanen Zelle und/oder einer neoplastischen Zelle oder einer Krebszelle.

7. In-Vitro-Verfahren zur Förderung von Selbsterneuerung und Hemmung der Differenzierung innerhalb einer Stammzelle, die nicht aus einem humanen Embryo stammt, welches die Hemmung der Bildung eines Blimp-1/Prmt5-Komplexes innerhalb der Stammzelle umfasst.

8. Verfahren nach Anspruch 7, wobei die Stammzelle ausgewählt ist aus: einer Säugetierstammzelle, einer humanen Stammzelle, einer adulten Stammzelle, einem Stammzellenvorläufer und einer pluripotenten Stammzelle.

9. Verfahren nach einem der Ansprüche 7 oder 8, wobei die Hemmung der Bildung eines Blimp-1/Prmt5-Komplexes innerhalb der Stammzelle erreicht wird, indem die Zelle mit einer Blimp-1-Inhibitorverbindung, einer Prmt5-Inhibitorverbindung und/oder einer Blimp-1/Prmt5-Komplex-Inhibitorverbindung in Kontakt gebracht wird.

10. Verfahren nach Anspruch 9, wobei die Inhibitorverbindung ausgewählt ist aus der Gruppe, welche aus einem kleinmolekularen Inhibitor; einem siRNA-Molekül, das an Blimp-1- oder Prmt5-mRNA bindet; einem Antisense-Oligonukleotid, das an Blimp-1- oder Prmt5-mRNA bindet, und einer dominanten negativen Version des Blimp-1- oder Prmt5-Polypeptids besteht.

11. Verfahren zur Identifizierung eines Blimp-1/Prmt5-Komplex-Antagonistenmoleküls, welches die Bestimmung, ob eine Verbindung an Blimp-1, Prmt5 und/oder den Blimp-1/Prmt5-Komplex bindet und dazu in der Lage ist, die biologische Aktivität oder Funktion von Blimp-1, Prmt5 und/oder dem Blimp-1/Prmt5-Komplex zu verringern, sowie die Identifikation einer solchen sich bindenden Verbindung als ein Molekül, welches mit dem Blimp-1/Prmt5-Komplex in Wechselwirkung tritt, umfasst und **dadurch gekennzeichnet ist, dass** eine Verbindung, die sich an Blimp-1, Prmt5 und/oder den Blimp-1/Prmt5-Komplex bindet und die biologische Aktivität oder Funktion von Blimp-1, Prmt5 und/oder dem Blimp-1/Prmt5-Komplex hemmt, als ein Antagonist des Blimp-1/Prmt5-Komplex identifiziert wird.

## Revendications

1. Complexe polypeptidique comprenant :
un premier domaine ayant une activité de liaison à l'ADN spécifique du site, dans lequel
le premier domaine se lie spécifiquement à un site de liaison consensus de type PRDI/Blimp1 de la protéine 1 de maturation induite par les lymphocytes B ; ou
le premier domaine comprend le polypeptide PRDI/Blimp1 ; ou
le premier domaine comprend une partie de liaison à l'ADN du polypeptide PRDI/Blimp1 ; et
un deuxième domaine ayant une activité arginine méthyltransférase qui effectue une déméthylation NG,N'G symétrique d'un résidu arginine et dans lequel l'activité arginine méthyltransférase comprend un domaine arginine méthyltransférase de la protéine arginine méthyltransférase 5 (Prmt5).

2. Construction d'un vecteur d'expression d'acides nucléiques, appropriée pour induire l'expression d'un complexe polypeptidique dans une cellule mammifère, le vecteur comprenant :
une ou plusieurs séquence(s) codante(s) fonctionnellement liée(s) à une séquence promotrice,
dans laquelle la ou les séquence(s) codante(s) codent pour au moins un premier domaine polypeptidique ayant une activité de liaison à l'ADN spécifique du site et au moins un deuxième domaine polypeptidique ayant une activité arginine méthyltransférase, dans laquelle
le premier domaine se lie spécifiquement à un site de liaison consensus de type PRDI/Blimp1 ; ou
le premier domaine comprend le polypeptide PRDI/Blimp1 ; ou
le premier domaine comprend une partie de liaison à l'ADN du polypeptide PRDI/Blimp1, et
le deuxième domaine a une activité arginine méthyltransférase qui effectue une déméthylation NG,N'G symétrique d'un résidu arginine situé dans un substrat polypeptidique et l'activité arginine méthyltransférase est comprise dans un domaine arginine méthyltransférase Prmt5.

3. Vecteur d'expression selon la revendication 2, dans lequel le substrat polypeptidique est une histone.

4. Procédé *in vitro* de contrôle de l'expression génétique dans une cellule mammifère comprenant l'induction de la formation dans la cellule d'un complexe polypeptidique comprenant au moins un premier domaine ayant une activité de liaison du site de liaison consensus de type PRDI/Blimp1 spécifique du site et au moins un deuxième domaine ayant une activité arginine méthyltransférase Prmt5.

5. Procédé selon la revendication 4, dans lequel la formation du complexe polypeptidique est induite dans la cellule par l'induction de l'expression d'un polypeptide PRDI/Blimp1 dans la cellule.

6. Procédé selon la revendication 4 ou 5, dans lequel la cellule mammifère est choisie parmi une cellule humaine ; et/ou une cellule néoplasique ou cancéreuse.

7. Procédé *in vitro* de stimulation du renouvellement automatique et d'inhibition de la différenciation dans une cellule souche ne provenant pas d'un embryon humain, comprenant l'inhibition de la formation d'un complexe Blimp1/Prmt5 dans la cellule souche.

8. Procédé selon la revendication 7, dans lequel la cellule souche est choisie parmi une cellule souche mammifère ; une cellule souche humaine ; une cellule souche adulte ; un progéniteur de cellules souches ; et une cellule souche pluripotente.

9. Procédé selon l'une quelconque des revendications 7 et 8, dans lequel l'inhibition de la formation d'un complexe Blimp1/Prmt5 dans la cellule souche est obtenue en exposant la cellule à un composé inhibiteur de Blimp1, un composé inhibiteur de Prmt5 et/ou un composé inhibiteur du complexe Blimp1/Prmt5.

10. Procédé selon la revendication 9, dans lequel le composé inhibiteur est choisi dans le groupe constitué par un inhibiteur de petites molécules ; une molécule ARNsi qui se lie à l'ARNm de Blimp1 ou Prmt5 ; un oligonucléotide antisens qui se lie à l'ARNm de Blimp1 ou Prmt5 ; et une version négative dominante du polypeptide Blimp1 ou Prmt5.

11. Procédé d'identification d'une molécule antagoniste du complexe Blimp1/Prmt5 comprenant la détermination de la liaison d'un composé et de sa capacité à modérer l'activité ou la fonction biologique de Blimp1, Prmt5 et/ou du complexe Blimp1/Prmt5, et l'identification de ce composé de liaison sous la forme d'une molécule d'interaction avec le complexe Blimp1/Prmt5, **caractérisé en ce qu'**un composé qui se lie et inhibe l'activité ou la fonction biologique de Blimp1, Prmt5 et/ou du complexe Blimp1/Prmt5, est identifié sous la forme d'un antagoniste du complexe Blimp1/Prmt5.
